# EUROPEAN PATENT SPECIFICATION

(11) **EP 3 376 884 B1**
(45) Date of publication and mention of the grant of the patent: **19.05.2021**
(21) Application number: 16784460.4
(22) Date of filing: 14.10.2016
(51) Int. Cl.: A24F 40/50

(54) **AEROSOL-GENERATING SYSTEM WITH SELF-ACTIVATED ELECTRIC HEATER**
AEROSOL-ERZEUGUNGSSYSTEM MIT SELBSTAKTIVIERTEM ELEKTRISCHEM HEIZKÖRPER
SYSTÈME DE GÉNÉRATION D'AÉROSOL AVEC CHAUFFAGE ÉLECTRIQUE AUTO-ACTIVÉ

(30) Priority: 17.11.2015 EP 15194990
(43) Date of publication of application: 26.09.2018
(73) Proprietor: Philip Morris Products S.A., 2000 Neuchâtel (CH)
(72) Inventor: REEVELL, Tony, London Greater London EC2A 4NE (GB)
(74) Representative: Ponder, William Anthony John
(86) International application number: PCT/EP2016/074798
(87) International publication number: WO 2017/084818

(56) References cited:
- EP-A1- 2 468 117
- US-A1- 2014 345 633

## Description

The present invention relates to an electrically operated aerosol-generating system. In particular, the present invention relates to an electrically operated aerosol-generating system in which an aerosol-forming substrate is liquid and is contained in a liquid storage portion.

WO 2012/085203 A1 or EP 2 468 117 A disclose an electrically heated smoking system having a liquid storage portion. The liquid storage portion includes a liquid aerosol-forming substrate and is connected to a vaporizer comprising an electric heater which is powered by a battery supply. In use, the electric heater is activated by suction on a mouthpiece by a user to switch on the battery power supply. The heated aerosol-forming substrate contained in the vaporizer will be vaporized. Suction on a mouthpiece by the user causes air to be drawn along or through the vaporizer thus generating an aerosol. The generated aerosol is drawn into the mouthpiece and subsequently into the mouth of a user. An amount of depletion of liquid aerosol-forming substrate is determined based on a relationship between a power applied to the heating element and a resulting temperature change of the heating element once the heating element is activated. The determined amount of depletion is indicated to the user.

This approach allows to determine the amount of depletion of liquid aerosol-forming substrate only when the electric heater is active. When using the electrically heated smoking system, the temperature of the active heater varies depending on the amount of concentration of liquid aerosol-forming substrate at the heating element. Furthermore, the concentration of liquid aerosol-forming substrate is affected by the amount of suction of a user. These possible variations have negative impact on the precision of the determined amount of depletion. In fact, the determined amount of depletion may not be estimated precise enough.

It would be desirable to provide an aerosol-generating system that determines the depletion before a user uses the aerosol-generating system and that improves accuracy of the determined amount of depletion.

According to a first aspect of the invention, there is provided an electrically operated aerosol-generating system for receiving an aerosol-forming substrate, the system comprising a liquid storage portion for storing liquid aerosol-forming substrate, an electric heater comprising at least one heating element for heating the liquid aerosol-forming substrate, and electric circuitry configured to self-activate the electric heater at a particular time for a self-activation duration in a period of inactivity of the electric heater in order to determine depletion of liquid aerosol-forming substrate based on a relationship between a power applied to the heating element and a resulting temperature change of the heating element.

The electric circuitry is preferably configured to estimate an amount of liquid aerosol-forming substrate in the liquid storage portion based on the determined depletion. The amount of liquid aerosol-forming substrate in the liquid storage portion may be an absolute amount or a relative amount, e.g. a percentage value, or may be a determination that there is more or less than a threshold amount of liquid aerosol-forming substrate in the liquid storage portion.

Self-activating the electric heater refers to activating the electric heater at a particular time when the electric heater is not in use, for example for the purpose of determining an amount of depletion of liquid aerosol-forming substrate.

Providing electric circuitry for self-activating the electric heater and determining depletion of liquid aerosol-forming substrate delivered to the heater is advantageous for a number of reasons. For example, when a user takes the aerosol-generating system, the depleted amount of liquid aerosol-forming substrate can be retrieved from the latest self-activation. Therefore, the user does not need to make a puff in order to activate the electric heater for retrieving the latest amount of depletion.

The electric heater is self-activated when a user does not require to generate aerosol, which may be the case when the aerosol-generating system is not in use, like a break between a series of puffs. Therefore, the present invention provides means to determine the consumption of liquid aerosol-forming substrate at advantageous points of time.

The consumption of liquid aerosol-forming substrate may be determined when the electric heater has cooled. The consumption of liquid aerosol-forming substrate may be determined when the concentration of the liquid aerosol-forming substrate at the heating element has reached a maximum value. In an aerosol-generating system comprising a wick as capillary medium to transport the liquid aerosol-forming substrate from the liquid storage portion to the heating element, the wick will draw the liquid aerosol-forming substrate until equilibrium is reached.

The consumption of liquid aerosol-forming substrate may be determined when the electric circuitry does not process another task or has a processing load below a threshold. Preferably, the consumption of liquid aerosol-forming substrate is determined when at least two, and more preferably all, of the aforementioned situations occur.

The self-activation of the electric heater allows to determine a level of liquid aerosol-forming substrate in the liquid storage portion before a user uses the aerosol-generating system again. This prevents the use of an aerosol-generating system when there are low levels of liquid aerosol-forming substrate, which is advantageous since heating with low levels of liquid aerosol-forming substrate can lead to overheating and potential problems resulting therefrom such as permanent damages of the aerosol-generating system.

With the self-activation of the electric heater, the determination of an amount of depletion can be controlled more precisely and does not need to be performed each time a user uses the aerosol-generating system. Advantageously, the level of liquid aerosol-forming substrate is determined independently from user activation. Power consumption may be limited by infrequent use of the self-activation.

During regular use of the aerosol-generating system, the electric circuitry may further be configured to activate the electric heater for a particular activation duration in response to a request for generating aerosol.

Preferably, the self-activation duration of the electric heater is shorter than an activation duration of the electric heater upon a user request for generating aerosol. Preferably, the self-activation duration is one of 0.1 seconds, 0.2 seconds, 0.3 seconds, 0.4 seconds, 0.5 seconds, 0.6 seconds, 0.7 seconds, 0.8 seconds, 0.9 seconds, and 1.0 second.

Preferably, the electric circuitry is configured to self-activate the electric heater only upon an occurrence of at least one self-activation precondition.

A first self-activation precondition may be a predetermined duration of inactivity of the electric heater. The self-activation may be performed once sufficient time has elapsed for the liquid aerosol-forming substrate to be replenished at the electric heater.

A second self-activation precondition may be a predetermined number of requested activations of the electric heater for generating aerosol.

A third self-activation precondition may occur when the temperature of the electric heater is below a minimum temperature threshold after a requested activation of the electric heater for generating aerosol.

A fourth self-activation precondition may occur when the concentration of the liquid aerosol-forming substrate at the heating element has reached a maximum value after a requested activation of the electric heater for generating aerosol.

Preferably, the electric circuitry is configured to self-activate the electric heater at least one more time in sequence in order to confirm the determined depletion of liquid aerosol-forming substrate of a previous measurement. The finally determined depletion may be an average value of the single estimates. A determined depletion may be confirmed by at least a second estimate that differs from the first estimate by a measurement error that is below a measurement error threshold.

Preferably, the electric circuitry is configured to estimate an amount of liquid aerosol-forming substrate in the liquid storage portion based on the determined depletion.

Preferably, the electric circuitry is configured to self-activate the electric heater more frequently the more the determined amount of liquid aerosol-forming substrate stored in the liquid storage portion decreases. Where it is determined that the level of liquid aerosol-forming substrate is getting to an empty state, the self-activations and resulting measurements may be taken more frequently. This enables the aerosol-generating system to use less power in determining the level of liquid aerosol-forming substrate when the liquid storage portion is rather full since it will not run out quickly. When the liquid storage portion gets to sensitive levels, more readings are taken so that the end of life for the liquid storage portion is not missed.

Preferably, the electric circuitry is configured to ignore requests for generating aerosol upon determining the volume of liquid aerosol-forming substrate stored in the liquid storage portion as being below a minimum volume threshold, thereby preventing activation of the electric heater.

Preferably, the aerosol-generating system further comprises a temperature sensor for measuring the temperature of the at least one heating element. The electric circuitry is arranged to monitor the temperature of the at least one heating element as sensed by the temperature sensor and determine depletion of liquid aerosol-forming substrate heated by the heater based on the temperature as sensed by the temperature sensor.

Preferably, the electric circuitry is arranged to measure the electrical resistance of the at least one heating element, to ascertain the temperature of the heating element from the measured electrical resistance.

Preferably, the electric circuitry is arranged to measure the electrical resistance of the at least one heating element by measuring the current through the at least one heating element and the voltage across the at least one heating element and determining the electrical resistance of the at least one heating element from the measured current and voltage.

The relationship between a temperature of the heating element and power applied to the heating element may be, for example, a rate of change of temperature of the heating element for a given power applied, an absolute temperature of the heating element at a given time during the self-activation for a given power applied, an integral of temperature over the self-activation duration for a given power applied or a power applied to the heating element in order to maintain a given temperature. In general terms, the less liquid aerosol-forming substrate is delivered to the heater for vaporisation, the higher the temperature of the heating element will be for a given applied power. For a given power, the evolution of the temperature of the heating element during the self-activation, and how that evolution changes over a plurality of self-activations, can be used to detect if there has been a depletion in the amount of aerosol-forming substrate delivered to the electric heater.

The electric heater comprises at least one heating element. The heating element may comprise an arrangement of filaments. Preferably, the at least one heating element is in the form of a heating wire or a coil or a filament encircling.

The at least one heating element preferably comprises an electrically resistive material. The at least one heating element may heat the liquid aerosol-forming substrate by means of conduction. The heating element may be at least partially in contact with the liquid aerosol-forming substrate. Alternatively, the heat from the heating element may be conducted to the liquid aerosol-forming substrate by means of a heat conductive element.

Preferably, the aerosol-generating system further comprises a capillary wick for conveying the liquid aerosol-forming substrate from the liquid storage portion to the electric heater. Preferably, the capillary wick is arranged to be in contact with liquid aerosol-forming substrate in the liquid storage portion. Preferably, the capillary wick extends into the liquid storage portion. In that case, in use, liquid is transferred from the liquid storage portion to the electric heater by capillary action in the capillary wick. The at least one heating element may support the capillary wick. The capillary properties of the wick, combined with the properties of the liquid, ensure that, during normal use when there is plenty of liquid aerosol-forming substrate, the wick is always wet in the heating area.

The aerosol-generating system may further comprise a temperature sensor for measuring the temperature of the at least one heating element when the heating element has been activated. The electric circuitry may be arranged to monitor the temperature of the at least one heating element as sensed by the temperature sensor and determine depletion of liquid aerosol-forming substrate heated by the heater based on the temperature of the at least one heating element as sensed by the temperature sensor.

If the amount of liquid aerosol-forming substrate has decreased, for example if the liquid storage portion is empty or nearly empty, insufficient liquid aerosol-forming substrate may be supplied to the heater. This may result in the temperature of the heating element increasing. Thus, the temperature of the heating element, as sensed by the temperature sensor, may allow the electric circuitry to determine that the amount of liquid aerosol-forming substrate in the liquid storage portion has decreased to a predetermined threshold and may further be able to provide an indication of an absolute amount of liquid aerosol-forming substrate in the liquid storage portion.

In a preferred embodiment, the electric circuitry is arranged to measure the electrical resistance of the at least one heating element, to ascertain the temperature of the heating element from the measured electrical resistance.

If the amount of liquid aerosol-forming substrate has decreased, for example if the liquid storage portion is empty or nearly empty, insufficient liquid aerosol-forming substrate may be supplied to the heater. This may result in the temperature of the heating element increasing. If the at least one heating element has suitable characteristics of the temperature coefficient of resistance, measuring the electrical resistance of the at least one heating element will allow the temperature of the heating element to be ascertained. Thus, the temperature of the heating element, as ascertained by the electric circuitry from the measured electrical resistance, may allow the electric circuitry to determine an amount of liquid aerosol-forming substrate in the liquid storage portion.

An advantage of this preferred latter embodiment is that it is not necessary to include a temperature sensor, which may take up valuable space in the aerosol-generating system and may also be costly. It is emphasized that the electrical resistance, in this embodiment, is used both as an 'actuator' (heating element) and a 'sensor' (temperature measurement).

In this preferred embodiment, the electric circuitry may be arranged to measure the electrical resistance of the at least one heating element by measuring the current through the at least one heating element and the voltage across the at least one heating element and determining the electrical resistance of the at least one heating element from the measured current and voltage. In that case, the electric circuitry may comprise a resistor, having a known resistance, in series with the at least one heating element and the electric circuitry may be arranged to measure the current through the at least one heating element by measuring the voltage across the known-resistance resistor and determining the current through the at least one heating element from the measured voltage and the known resistance. The electric circuitry may be arranged to determine depletion of liquid aerosol-forming substrate heated by the heater by monitoring an increase of the sensed or ascertained temperature over successive heating cycles as the liquid aerosol-forming substrate in the liquid storage portion is consumed.

The electric circuitry may be arranged to determine depletion of liquid aerosol-forming substrate heated by the heater by monitoring the rate of increase of the sensed or ascertained temperature at the start of a self-activation of the electric heater, over successive self-activations of the electric heater while the liquid aerosol-forming substrate in the liquid storage portion is consumed between the self-activations of the electric heater.

The electric circuitry may be arranged to determine an amount of liquid aerosol-forming substrate in the liquid storage portion by monitoring an increase in the value of an integral over time of the sensed or ascertained temperature over the self-activation duration of the electric heater.

In a preferred embodiment, the electric circuitry is arranged, when the amount of liquid aerosol-forming substrate in the liquid storage portion is estimated to have decreased to a predetermined threshold, to deactivate the electric heater.

This is advantageous because the user can then no longer use the aerosol-generating system once there is insufficient liquid aerosol-forming substrate. This will avoid creation of an aerosol which does not have the desired properties. This will avoid a poor experience for the user.

The electric circuitry may be arranged to deactivate the electric heater either permanently or temporarily until conditions have changed that allow further operation of the electric heater. The electric circuitry may deactivate the electric heater permanently by blowing an electrical fuse between the electric heater and an electric power supply. The electric circuitry may be arranged to deactivate the electric heater temporarily by switching off a switch between the electric heater and an electric power supply. As soon as conditions have changed that allow further operation of the electric heater, for example after refilling an empty liquid storage portion or after replacing a depleted liquid storage portion by new one, the switch deactivating the electric heater may be turned on again.

In a preferred embodiment, the electric circuitry is arranged, when the amount of liquid aerosol-forming substrate in the liquid storage portion is estimated to have decreased to the predetermined threshold, to indicate this to a user. This is advantageous because the indication enables the user to refill or replace the liquid storage portion.

The electrically operated aerosol-generating system may comprise a user display. In that case, the indication may comprise an indication on the user display. Alternatively, the indication may comprise an audible indication, or any other suitable type of indication for a user.

For allowing ambient air to enter the aerosol-generating system, a wall of the housing of the aerosol-generating system, preferably a wall opposite the electric heater, preferably a bottom wall, is provided with at least one semi-open inlet. The semi-open inlet allows air to enter the aerosol-generating system, but no air or liquid to leave the aerosol-generating system through the semi-open inlet. A semi-open inlet may for example be a semi-permeable membrane, permeable in one direction only for air, but is air- and liquid-tight in the opposite direction. A semi-open inlet may for example also be a one-way valve. Preferably, the semi-open inlets allow air to pass through the inlet only if specific conditions are met, for example a minimum depression in the aerosol-generating system or a volume of air passing through the valve or membrane.

The liquid aerosol-forming substrate is a substrate capable of releasing volatile compounds that can form an aerosol. The volatile compounds may be released by heating the liquid aerosol-forming substrate. The liquid aerosol-forming substrate may comprise plant-based material. The liquid aerosol-forming substrate may comprise tobacco. The liquid aerosol-forming substrate may comprise a tobacco-containing material containing volatile tobacco flavour compounds, which are released from the liquid aerosol-forming substrate upon heating. The liquid aerosol-forming substrate may alternatively comprise a non-tobacco-containing material. The liquid aerosol-forming substrate may comprise homogenised plant-based material. The liquid aerosol-forming substrate may comprise homogenised tobacco material. The liquid aerosol-forming substrate may comprise at least one aerosol-former. The liquid aerosol-forming substrate may comprise other additives and ingredients, such as flavourants.

An advantage of providing a liquid storage portion is that the liquid aerosol-forming substrate in the liquid storage portion is protected from ambient air. In some embodiments, ambient light cannot enter the liquid storage portion as well, so that the risk of light-induced degradation of the liquid aerosol-forming substrate is avoided. Moreover, a high level of hygiene can be maintained.

Preferably, the liquid storage portion is arranged to hold liquid aerosol-forming substrate for a predetermined number of puffs. If the liquid storage portion is not refillable and the liquid in the liquid storage portion has been used up, the liquid storage portion has to be replaced by the user. During such replacement, contamination of the user with liquid aerosol-forming substrate has to be prevented. Alternatively, the liquid storage portion may be refillable. In that case, the aerosol-generating system may be replaced after a certain number of refills of the liquid storage portion.

The aerosol-generating system advantageously comprises a power supply, typically a battery, within the main body of the housing. As an alternative, the power supply may be another form of charge storage device such as a capacitor. The power supply may require recharging and may have a capacity that allows for the storage of enough energy for one or more smoking experiences; for example, the power supply may have sufficient capacity to allow for the continuous generation of aerosol for a period of around six minutes or for a period that is a multiple of six minutes. In another example, the power supply may have sufficient capacity to allow for a predetermined number of puffs or discrete activations of the heater assembly.

The aerosol-generating system may comprise a main unit and a cartridge that is removably coupled to the main unit, wherein the liquid storage portion and the electric heater are provided in the cartridge and the main unit comprises a power supply and the electric circuitry.

The aerosol-generating system is electrically operated and may be an electrically operated smoking system. Preferably, the aerosol-generating system is portable. The aerosol-generating system may have a size comparable to a conventional cigar or cigarette. The smoking system may have a total length between approximately 30 millimeter and approximately 150 millimeter. The smoking system may have an external diameter between approximately 5 millimeter and approximately 30 millimeter.

According to a second aspect of the invention, there is provided a method comprising providing an electrically operated aerosol-generating system comprising a liquid storage portion for storing liquid aerosol-forming substrate and an electric heater comprising at least one heating element for heating the liquid aerosol-forming substrate, self-activating the electric heater at a particular time and for a self-activation duration in a period of inactivity of the electric heater, and determining depletion of liquid aerosol-forming substrate heated by the electric heater based on a relationship between a power applied to the heating element and a resulting temperature change of the heating element.

The amount of liquid aerosol-forming substrate may be an absolute amount or a relative amount, for example a percentage value, or may be a determination that there is more or less than a threshold amount of liquid aerosol-forming substrate in the liquid storage portion.

According to a third aspect of the invention, there is provided electric circuitry for an electrically operated aerosol-generating system, the electric circuitry being arranged to perform the method of the second aspect of the invention.

The electric circuitry is configured to regulate a supply of power to the electric heater. Power is supplied to the electric heater continuously following activation of the system or may be supplied intermittently, such as on a puff-by-puff basis. The power may be supplied to the electric heater in the form of pulses of electrical current.

The electric circuitry may comprise a microprocessor, which may be a programmable microprocessor being arranged to perform the method of the second aspect of the invention. The electric circuitry may comprise further electronic components.

According to a fourth aspect of the invention, there is provided a computer readable storage medium having stored thereon a computer program which, when run on programmable electric circuitry for an electrically operated aerosol-generating system, causes the programmable electric circuitry to perform the method of the second aspect of the invention.

Features described in relation to one aspect may equally be applied to other aspects of the invention. Preferable features may be combined with other preferable features. This also applies to features described in relation to the aerosol-generating system of the invention which may be applicable to the method of the invention. Features described in relation to the method of the invention may also be applicable to the aerosol-generating system of the invention.

The invention will be further described, by way of example only, with reference to the accompanying drawings, of which:
Figure 1 is a plot showing coil activations in response to user requests for generating aerosol and showing self-activations of the coil over time according to a first embodiment;
Figure 2 is a plot showing coil activations in response to user requests for generating aerosol and showing self-activations of the coil over time according to a second embodiment;
Figure 3 is a plot showing five medians of temperature profiles of the heating element during multiple puffs of an electrically operated aerosol-generating system; and
Figure 4 shows one example of an electrically operated aerosol-generating system having a liquid storage portion.

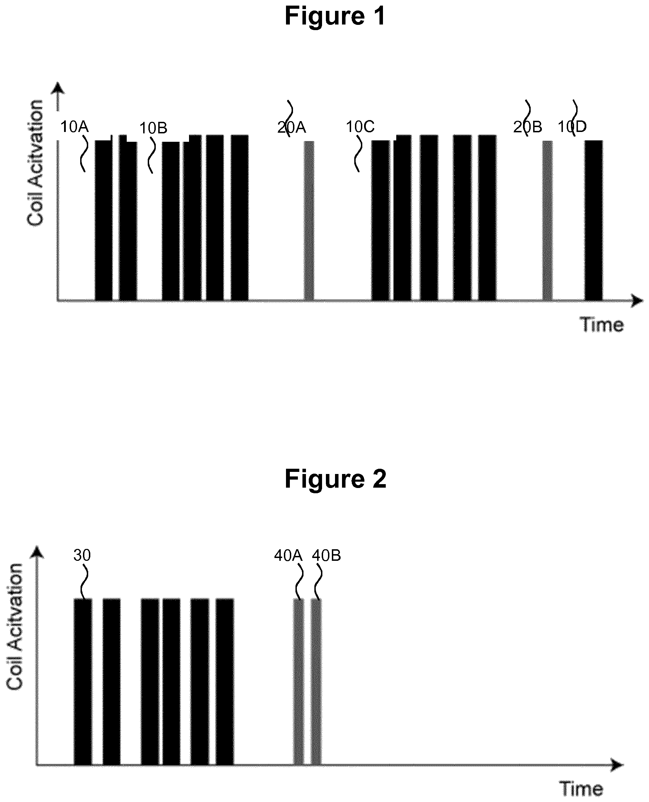

Figure 1 shows coil activations over time according to a first embodiment of an aerosol-generating system comprising a heating coil and a wick transporting liquid aerosol-forming substrate from the liquid storage portion to the electric heater. The plot indicates the time and duration of single coil activations. The coil may either be activated in response to a user request for generating aerosol, for example by making a puff, or by a self-activation of the electric heater in a period of inactivity of the electric heater. Coil activation 10A is the first activation of the electric heater resulting from a first series of puffs. Coil activation 10B represents the first puff of a second series of puffs that occurs after a break. The break was short enough that no self-activation of the electric heater could take place between the first and second series of puffs initiated by coil activations 10A and 10B. After the second series of puffs, a break follows that is sufficiently long for a self-activation 20A of the coil. After a while, the user uses the aerosol-generating system again and continues with a third series of puffs starting with coil activation 10C. Again, a break follows that is sufficiently long for another self-activation 20B of the coil.

The plot in Figure 1 shows that self-activations of the electric heater only occur when a user has not puffed on the aerosol-generating system for a given time. Preferably, the time interval is selected such that the coil has cooled and the liquid wicking has reached an equilibrium. The self-activations of the electric heater are much shorter in duration than the user activations of the electric heater. The self-activations occur after a certain period of inactivity, after a user activation session. Therefore, the infrequent self-activation does not have a large impact on the battery life if the power supply is realized by a battery.

Figure 2 shows coil activations over time according to a second embodiment of an aerosol-generating system comprising a heating coil and a wick transporting liquid aerosol-forming substrate from the liquid storage portion to the electric heater. After a series of six puffs which are initiated by a first coil activation 30, a break occurs that is sufficiently long that the electric heater is self-activated 40A for the first time. In the course of the self-activation of the electric heater, the depleted amount of liquid aerosol-forming is determined. In order to confirm the determined depletion, a second self-activation 40B of the electric heater is initiated to determine the depletion again. In the given example, the difference between the second determined amount and the first determined amount is below an measurement error threshold so that the result is confirmed.

This approach improves reliability of the measurements and is especially useful when determining that the liquid aerosol-forming substrate is depleted and a cartridge comprising the liquid storage portion should not be used any longer. Under these circumstances, the electric circuitry of the aerosol-generating system may prohibit the further use of the aerosol-generating system. Prior to prohibiting the further use, it is advantageous to confirm the determined amount by a second measurement a short time after the first measurement to avoid an erroneously deactivation of the aerosol-generating system caused by a single false detection of an empty cartridge.

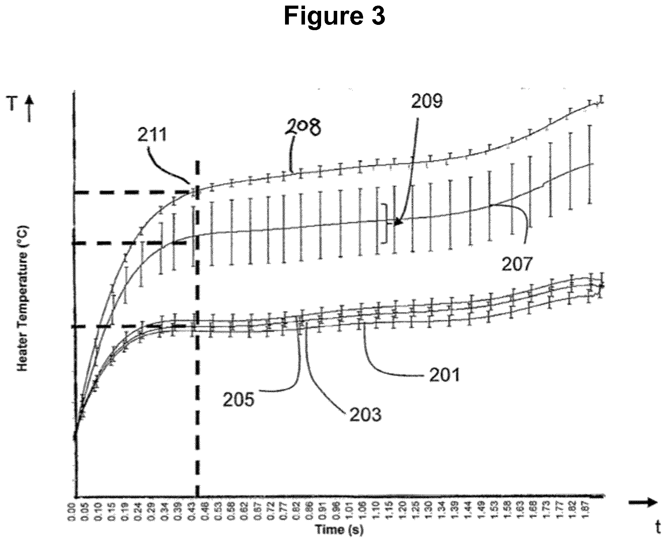

Figure 3 is a plot showing five medians of temperature profiles being measured during multiple puffs of an aerosol-generating system when the electric heater is activated because of a user request for generating aerosol. The temperature T of the heating element is shown on the y-axis and the puff time t is shown on the x-axis. Curve 201 is the median of a first set of puffs, each puff having a 2-second puff duration. Similarly, curve 203 is the median of a second set of puffs, curve 205 is the median of a third set of puffs, curve 207 is the median of a fourth set of puffs and curve 208 is the median of a fifth set of puffs. In each curve, the vertical bars (for example shown at 209) indicate the standard deviation around the median for those temperatures. Thus, the evolution of the measured temperature over the life of the liquid storage portion is shown. This behaviour was observed and confirmed for all liquid formulations vaporized and for all power levels used.

As can be seen from Figure 3, the temperature response of the heating element is reasonably stable over curves 201, 203 and 205. That is to say, the standard deviation around the median for the first three sets of puffs is reasonably small. Over curve 207, two effects are noticed. Firstly, the standard deviation around the median for the third set of puffs is greater. Secondly, the temperature of the heating element during each puff has significantly increased. These two effects indicate that the liquid storage portion is becoming empty.

Over curve 208, the standard deviation around the median for the fifth set of puffs is smaller once again. That is to say, the temperature range over the puffs is reasonably stable. However, the temperature of the heating element during each puff has increased further. This indicates that the liquid storage portion is substantially empty.

The temperature increase in curve 207, as compared with curve 205, is particularly evident after around 0.4 seconds of the puff (shown by dotted line 211). Detecting that the amount of liquid in the liquid storage portion has decreased to a threshold can therefore be accurately based on the temperature level of the heating element after 0.4 seconds of the puff duration.

Empirical data for particular designs of aerosol-forming substrate and for the particular system design can be stored in memory in the electric circuitry. This empirical data can relate the temperature of the heating element at a particular point in a puff or heating cycle operating at a given power with the amount of liquid remaining in the liquid storage portion. The empirical data can then be used to determine how much liquid is remaining and may be used to provide a user with an indication when there is estimated to be less than a predetermined number of puffs remaining.

Thus, Figure 3 demonstrates that there is a clear temperature increase of the heating element as the liquid storage portion becomes empty. This is particularly evident after the first 0.4 seconds of a puff. This temperature increase can be utilized to determine when the liquid storage portion is empty or nearly empty.

It can also be seen in Figure 3 that the slope of the temperature profile between 0 seconds and 0.2 seconds increases as the liquid storage portion becomes empty. Thus, a measure of the rate of temperature increase during an initial time of a puff over the life of the liquid storage portion can provide an alternative or additional means to detect an amount of the remaining liquid in the liquid storage portion.

Due to these results, the duration of the self-activation of the electric heater performed during a period of inactivity of the electric heater may be shorter than the activation duration of the electric heater during a puff. When determining the amount of depletion during a self-activation of the electric heater, faster insight into the temperature level change may be given, thereby reducing the risk of poor aerosol properties.

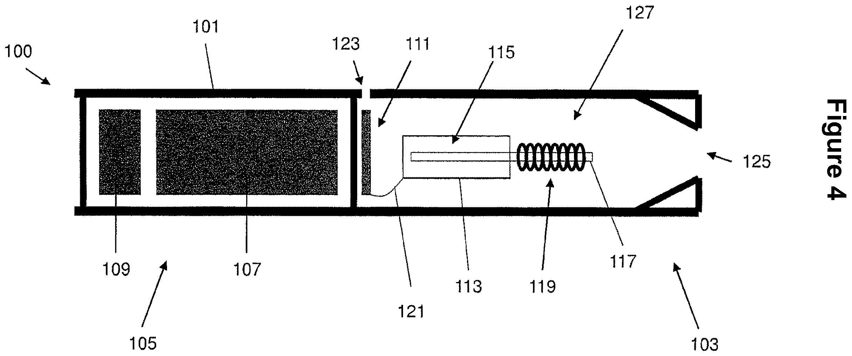

Figure 4 shows one example of an aerosol-generating system having a liquid storage portion. In Figure 4, the system is a smoking system. The smoking system 100 of Figure 4 comprises a housing 101 having a mouthpiece end 103 and a body end 105. In the body end, there is provided an electric power supply in the form of battery 107 and electric circuitry 109. A puff detection system 111 is also provided in cooperation with the electric circuitry 109. In the mouthpiece end, there is provided a liquid storage portion in the form of cartridge 113 containing liquid aerosol-forming substrate 115, a capillary wick 117 and an electric heater 119. Note that the electric heater is only shown schematically in Figure 4. In the exemplary embodiment shown in Figure 4, one end of capillary wick 117 extends into cartridge 113, and the other end of capillary wick 117 is surrounded by the electric heater 119. The electric heater 119 is connected to the electric circuitry via connections 121, which may pass along the outside of cartridge 113 (not shown in Figure 4). The housing 101 also includes an air inlet 23, an air outlet 125 at the mouthpiece end, and an aerosol-forming chamber 127.

In use, operation is as follows. Liquid aerosol-forming substrate 115 is conveyed by capillary action from the cartridge 113 from the end of the wick 117 which extends into the cartridge to the other end of the wick which is surrounded by the electric heater 119. When a user draws on the aerosol-generating system at the air outlet 125, ambient air is drawn through air inlet 123. In the arrangement shown in Figure 4, the puff detection system 111 senses the puff and activates the electric heater 119. The battery 107 supplies electrical energy to the heater 119 to heat the end of the wick 117 surrounded by the electric heater. The liquid in that end of the wick 117 is vaporized by the electric heater 119 to create a supersaturated vapour. At the same time, the liquid aerosol-forming substrate being vaporized is replaced by further liquid moving along the wick 117 by capillary action. (This is sometimes referred to as "pumping action".) The supersaturated vapour created is mixed with and carried in the air flow from the air inlet 123. In the aerosol-forming chamber 127, the vapour condenses to form an inhalable aerosol, which is carried towards the outlet 125 and into the mouth of the user.

In the embodiment shown in Figure 4, the electric circuitry 109 and puff detection system 111 are preferably programmable. The electric circuitry 109 and puff detection system 111 can be used to manage operation of the aerosol-generating system. This assists with control of the particle size in the aerosol.

Figure 4 shows one example of an aerosol-generating system according to the present invention. Many other examples are possible, however. In addition, note that Figure 4 is schematic in nature. In particular, the components shown are not to scale either individually or relative to one another. The aerosol-generating system needs to include or receive a liquid aerosol-forming substrate contained in a liquid storage portion. The aerosol-generating system requires some sort of electric heater having at least one heating element for heating the liquid aerosol-forming substrate. Finally, the aerosol-generating system requires electric circuitry for self-activating the electric heater at a particular time and for a self-activation duration in a period of inactivity of the electric heater in order to determine depletion of liquid aerosol-forming substrate in the liquid storage portion. For example, the system need not be a smoking system. A puff detection system need not be provided. Instead, the system could operate by manual activation, for example the user operating a switch when a puff is taken. For example, the overall shape and size of the housing could be altered. Moreover, the system may not include a capillary wick. In that case, the system may include another mechanism for delivering liquid for vaporization.

The exemplary embodiments described above illustrate but are not limiting. In view of the above discussed exemplary embodiments, other embodiments consistent with the above exemplary embodiments will now be apparent to one of ordinary skill in the art.

## Claims

1. An electrically operated aerosol-generating system for receiving an aerosol-forming substrate (115), the system comprising:
a liquid storage portion for storing liquid aerosol-forming substrate;
an electric heater (119) comprising at least one heating element for heating the liquid aerosol-forming substrate; and
electric circuitry (109) configured to self-activate the electric heater at a particular time for a self-activation duration in a period of inactivity of the electric heater in order to determine depletion of liquid aerosol-forming substrate based on a relationship between a power applied to the heating element and a resulting temperature change of the heating element.

2. An electrically operated aerosol-generating system according to claim 1, wherein the electric circuitry (109) is further configured to activate the electric heater (119) for a particular activation duration in response to a request for generating aerosol.

3. An electrically operated aerosol-generating system according to claim 2, wherein the self-activation duration of the electric heater (109) is shorter than an activation duration of the electric heater for generating aerosol.

4. An electrically operated aerosol-generating system according to any of claims 1 to 3, wherein the electric circuitry (109) is configured to self-activate the electric heater (119) only upon an occurrence of at least one self-activation precondition.

5. An electrically operated aerosol-generating system according to claim 4, wherein one of the at least one self-activation precondition is a predetermined duration of inactivity of the electric heater (119), or
wherein one of the at least one self-activation precondition is a predetermined number of requested activations of the electric heater for generating aerosol, or
wherein one of the at least one self-activation precondition occurs when the temperature of the electric heater is below a minimum temperature threshold after a requested activation of the electric heater for generating aerosol, or
wherein one of the at least one self-activation precondition occurs when the concentration of the liquid aerosol-forming substrate (115) at the heating element has reached a maximum value after a requested activation of the electric heater for generating aerosol.

6. An electrically operated aerosol-generating system according to any of claims 1 to 5, wherein the electric circuitry (109) is configured to self-activate the electric heater (119) at least one more time in sequence in order to confirm the determined depletion of liquid aerosol-forming substrate (115) of a previous measurement.

7. An electrically operated aerosol-generating system according to any of claims 1 to 6, wherein the electric circuitry (109) is configured to estimate an amount of liquid aerosol-forming substrate (115) in the liquid storage portion based on the determined depletion.

8. An electrically operated aerosol-generating system according to any of claim 1 to 7, wherein the electric circuitry (109) is configured to self-activate the electric heater (119) more frequently the more the determined amount of liquid aerosol-forming substrate (115) stored in the liquid storage portion decreases.

9. An electrically operated aerosol-generating system according to any of claims 1 to 8, wherein the electric circuitry (109) is configured to ignore requests for generating aerosol upon determining the volume of liquid aerosol-forming substrate stored in the liquid storage portion as being below a minimum volume threshold, thereby preventing activation of the electric heater (119).

10. An electrically operated aerosol-generating system according to any of claims 1 to 9, further comprising a temperature sensor for measuring the temperature of the at least one heating element and wherein the electric circuitry is arranged to monitor the temperature of the at least one heating element as sensed by the temperature sensor and determine depletion of liquid aerosol-forming substrate (115) heated by the heater based on the temperature as sensed by the temperature sensor.

11. An electrically operated aerosol-generating system according to any of claims 1 to 10, wherein the electric circuitry (109) is arranged to measure the electrical resistance of the at least one heating element, to ascertain the temperature of the heating element from the measured electrical resistance.

12. An electrically operated aerosol-generating system according to claim 11, wherein the electric circuitry (109) is arranged to measure the electrical resistance of the at least one heating element by measuring the current through the at least one heating element and the voltage across the at least one heating element and determining the electrical resistance of the at least one heating element from the measured current and voltage.

13. A method comprising:
providing an electrically operated aerosol-generating system comprising a liquid storage portion for storing liquid aerosol-forming substrate (115) and an electric heater (119) comprising at least one heating element for heating the liquid aerosol-forming substrate;
self-activating the electric heater at a particular time for a self-activation duration in a period of inactivity of the electric heater; and
determining depletion of liquid aerosol-forming substrate heated by the electric heater based on a relationship between a power applied to the heating element and a resulting temperature change of the heating element.

14. Electric circuitry for an electrically operated aerosol-generating system comprising a liquid storage portion for storing liquid aerosol-forming substrate (115) and an electric heater (119) comprising at least one heating element for heating the liquid aerosol-forming substrate, the electric circuitry (109) being arranged to perform the method of claim 13.

15. A computer readable storage medium having stored thereon a computer program which, when run on programmable electric circuitry for an electrically operated aerosol-generating system comprising a liquid storage portion for storing liquid aerosol-forming substrate (115) and an electric heater (119) comprising at least one heating element for heating the liquid aerosol-forming substrate, causes the programmable electric circuitry to perform the method of claim 13.

## Patentansprüche

1. Elektrisch betriebenes Aerosolerzeugungssystem zum Aufnehmen eines aerosolbildenden Substrats (115), wobei das System aufweist:
einen Flüssigspeicherteil zum Speichern von flüssigem aerosolbildendem Substrat;
eine elektrische Heizvorrichtung (119), die mindestens ein Heizelement zum Erwärmen des flüssigen aerosolbildenden Substrats aufweist; und
elektrische Schaltkreise (109), die so ausgelegt sind, dass sie die elektrische Heizvorrichtung zu einem bestimmten Zeitpunkt für eine Selbstaktivierungsdauer in einem Zeitraum der Inaktivität der elektrischen Heizvorrichtung selbst aktivieren, um die Entleerung von flüssigem aerosolbildendem Substrat basierend auf einer Beziehung zwischen einer Energie, die an das Heizelement bereitgestellt wird, und einer resultierenden Temperaturänderung des Heizelements zu ermitteln.

2. Elektrisch betriebenes Aerosolerzeugungssystem nach Anspruch 1, wobei elektrische Schaltkreise (109) ferner so ausgelegt sind, dass sie die elektrische Heizvorrichtung (119) für eine bestimmte Aktivierungsdauer in Reaktion auf eine Anforderung zum Erzeugen von Aerosol aktivieren.

3. Elektrisch betriebenes Aerosolerzeugungssystem nach Anspruch 2, wobei die Selbstaktivierungsdauer der elektrischen Heizvorrichtung (109) kürzer ist als eine Aktivierungsdauer der elektrischen Heizvorrichtung zum Erzeugen von Aerosol.

4. Elektrisch betriebenes Aerosolerzeugungssystem nach einem der Ansprüche 1 bis 3, wobei die elektrischen Schaltkreise (109) so ausgelegt sind, dass sie die elektrische Heizvorrichtung (119) nur bei Auftreten mindestens einer Selbstaktivierungsvoraussetzung selbst aktivieren.

5. Elektrisch betriebenes Aerosolerzeugungssystem nach Anspruch 4, wobei eine der mindestens einen Selbstaktivierungsvoraussetzung eine vorbestimmte Dauer der Inaktivität der elektrischen Heizvorrichtung (119) ist, oder
wobei eine der mindestens einen Selbstaktivierungsvoraussetzung eine vorbestimmte Anzahl an angeforderten Aktivierungen der elektrischen Heizvorrichtung zum Erzeugen von Aerosol ist, oder
wobei eine der mindestens einen Selbstaktivierungsvoraussetzung auftritt, wenn die Temperatur der elektrischen Heizvorrichtung nach einer angeforderten Aktivierung der elektrischen Heizvorrichtung zum Erzeugen von Aerosol unter einer Mindesttemperaturschwelle liegt, oder
wobei eine der mindestens einen Selbstaktivierungsvoraussetzung auftritt, wenn die Konzentration des flüssigen aerosolbildenden Substrats (115) an dem Heizelement nach einer angeforderten Aktivierung der elektrischen Heizvorrichtung zum Erzeugen von Aerosol einen Maximalwert erreicht hat.

6. Elektrisch betriebenes Aerosolerzeugungssystem nach einem der Ansprüche 1 bis 5, wobei die elektrischen Schaltkreise (109) so ausgelegt sind, dass sie die elektrische Heizvorrichtung (119) mindestens ein weiteres Mal der Reihe nach selbst aktivieren, um die ermittelte Entleerung des flüssigen aerosolbildenden Substrats (115) von einer vorherigen Messung zu bestätigen.

7. Elektrisch betriebenes Aerosolerzeugungssystem nach einem der Ansprüche 1 bis 6, wobei die elektrischen Schaltkreise (109) so ausgelegt sind, dass sie eine Menge an flüssigem aerosolbildendem Substrat im Flüssigspeicherteil (115) basierend auf der ermittelten Entleerung abschätzen.

8. Elektrisch betriebenes Aerosolerzeugungssystem nach einem der Ansprüche 1 bis 7, wobei die elektrischen Schaltkreise (109) so ausgelegt sind, dass sie die elektrische Heizvorrichtung (119) umso häufiger selbst aktivieren, desto mehr die ermittelte Menge an flüssigem aerosolbildendem Substrat, das im Flüssigspeicherteil (115) gespeichert ist, abnimmt.

9. Elektrisch betriebenes Aerosolerzeugungssystem nach einem der Ansprüche 1 bis 8, wobei die elektrischen Schaltkreise (109) ausgelegt sind, um Anforderungen zur Erzeugung von Aerosol bei der Ermittlung des Volumens des flüssigen aerosolbildenden Substrats, das in dem Flüssigspeicherteil gespeichert ist, als unter einer minimalen Volumenschwelle zu ignorieren, wodurch eine Aktivierung der elektrischen Heizvorrichtung (119) verhindert wird.

10. Elektrisch betriebenes Aerosolerzeugungssystem nach einem der Ansprüche 1 bis 9, ferner aufweisend einen Temperatursensor zum Messen der Temperatur von dem mindestens einen Heizelement, und wobei die elektrischen Schaltkreise angeordnet sind, um die Temperatur von dem mindestens einen Heizelement, wie erfasst durch den Temperatursensor, zu überwachen und die Entleerung von durch die Heizvorrichtung erwärmtem flüssigem aerosolbildendem Substrat (115) basierend auf der Temperatur, wie erfasst durch den Temperatursensor, zu ermitteln.

11. Elektrisch betriebenes Aerosolerzeugungssystem nach einem der Ansprüche 1 bis 10, wobei die elektrischen Schaltkreise (109) angeordnet sind, um den elektrischen Widerstand von dem mindestens einen Heizelement zu messen, um die Temperatur des Heizelements von dem gemessenen elektrischen Widerstand zu ermitteln.

12. Elektrisch betriebenes Aerosolerzeugungssystem nach Anspruch 11, wobei die elektrischen Schaltkreise (109) angeordnet sind, um den elektrischen Widerstand von dem mindestens einen Heizelement durch Messen des Stroms durch das mindestens eine Heizelement und der Spannung über dem mindestens einen Heizelement und Ermitteln des elektrischen Widerstandes des mindestens einen Heizelements von dem gemessenen Strom und der gemessenen Spannung zu messen.

13. Verfahren, aufweisend:
Vorsehen eines elektrisch betriebenen Aerosolerzeugungssystems, das einen Flüssigspeicherteil zum Speichern von flüssigem aerosolbildendem Substrat (115) und eine elektrische Heizvorrichtung (119) aufweist, die mindestens ein Heizelement zum Erwärmen des flüssigen aerosolbildenden Substrats aufweist;
Selbstaktivierung der elektrischen Heizvorrichtung zu einer bestimmten Zeit für eine Selbstaktivierungsdauer in einem Zeitraum der Inaktivität der elektrischen Heizvorrichtung; und
Ermitteln der Entleerung des flüssigen aerosolbildenden Substrats, das durch die elektrische Heizvorrichtung erwärmt wird, basierend auf einer Beziehung zwischen einer Energie, die an das Heizelement bereitgestellt wird, und einer resultierenden Temperaturänderung des Heizelements.

14. Elektrische Schaltkreise für ein elektrisch betriebenes Aerosolerzeugungssystem, das einen Flüssigspeicherteil zum Speichern des flüssigen aerosolbildenden Substrats (115) und eine elektrische Heizvorrichtung (119) aufweist, die mindestens ein Heizelement zum Erwärmen des flüssigen aerosolbildenden Substrats aufweist, und die elektrischen Schaltkreise (109) angeordnet sind, um das Verfahren nach Anspruch 13 auszuführen.

15. Computerlesbares Speichermedium, auf dem ein Computerprogramm gespeichert ist, das, bei Ausführung auf programmierbaren elektrischen Schaltkreisen für ein elektrisch betriebenes Aerosolerzeugungssystem, das einen Flüssigspeicherteil zum Speichern des flüssigen aerosolbildenden Substrats (115) und eine elektrische Heizvorrichtung (119) aufweist, die mindestens ein Heizelement zum Erwärmen des flüssigen aerosolbildenden Substrats aufweist, veranlasst, die programmierbaren elektrischen Schaltkreise das Verfahren nach Anspruch 13 auszuführen.

## Revendications

1. Système de génération d'aérosol à fonctionnement électrique pour la réception d'un substrat formant aérosol (115), le système comprenant :
une partie de stockage de liquide pour stocker un substrat formant aérosol liquide ;
un dispositif de chauffage électrique (119) comprenant au moins un élément de chauffage pour chauffer le substrat formant aérosol liquide ; et
des circuits électriques (109) configurés pour activer automatiquement le dispositif de chauffage électrique à un moment particulier pendant une durée d'activation automatique dans une période d'inactivité du dispositif de chauffage électrique afin de déterminer l'épuisement du substrat formant aérosol liquide sur la base d'une relation entre une puissance appliquée à l'élément de chauffage et une variation de température résultante de l'élément de chauffage.

2. Système de génération d'aérosol à fonctionnement électrique selon la revendication 1, dans lequel les circuits électriques (109) sont en outre configurés pour activer le dispositif de chauffage électrique (119) pendant une durée d'activation particulière en réponse à une demande de génération d'aérosol.

3. Système de génération d'aérosol à fonctionnement électrique selon la revendication 2, dans lequel la durée d'activation automatique du dispositif de chauffage électrique (109) est plus courte qu'une durée d'activation du dispositif de chauffage électrique pour générer un aérosol.

4. Système de génération d'aérosol à fonctionnement électrique selon l'une quelconque des revendications 1 à 3, dans lequel les circuits électriques (109) sont configurés pour activer automatiquement le dispositif de chauffage électrique (119) uniquement lors d'une occurrence d'au moins une condition préalable d'activation automatique.

5. Système de génération d'aérosol à fonctionnement électrique selon la revendication 4, dans lequel l'une parmi l'au moins une condition préalable d'activation automatique est une durée prédéterminée d'inactivité du dispositif de chauffage électrique (119), ou
dans lequel l'une parmi l'au moins une condition préalable d'activation automatique est un nombre prédéterminé d'activations demandées du dispositif de chauffage électrique pour générer un aérosol, ou
dans lequel l'une parmi l'au moins une condition préalable d'activation automatique se produit lorsque la température du dispositif de chauffage électrique est inférieure à un seuil de température minimal après une activation demandée du dispositif de chauffage électrique pour générer un aérosol, ou
dans lequel l'une parmi l'au moins une condition préalable d'activation automatique se produit lorsque la concentration du substrat formant aérosol liquide (115) au niveau de l'élément de chauffage a atteint une valeur maximale après une activation demandée du dispositif de chauffage électrique pour générer un aérosol.

6. Système de génération d'aérosol à fonctionnement électrique selon l'une quelconque des revendications 1 à 5, dans lequel les circuits électriques (109) sont configurés pour activer automatiquement le dispositif de chauffage électrique (119) au moins une fois de plus en séquence afin de confirmer l'épuisement déterminé du substrat formant aérosol liquide (115) d'une mesure précédente.

7. Système de génération d'aérosol à fonctionnement électrique selon l'une quelconque des revendications 1 à 6, dans lequel les circuits électriques (109) sont configurés pour estimer une quantité de substrat formant aérosol liquide (115) dans la partie de stockage de liquide sur base de l'épuisement déterminé.

8. Système de génération d'aérosol à fonctionnement électrique selon l'une quelconque des revendications 1 à 7, dans lequel les circuits électriques (109) sont configurés pour activer automatiquement le dispositif de chauffage électrique (119) plus fréquemment plus la quantité déterminée de substrat formant aérosol liquide (115) stockée dans la partie de stockage de liquide diminue.

9. Système de génération d'aérosol à fonctionnement électrique selon l'une quelconque des revendications 1 à 8, dans lequel les circuits électriques (109) sont configurés pour ignorer les demandes de génération d'aérosol lorsqu'il est déterminé que le volume du substrat formant aérosol liquide stocké dans la partie de stockage de liquide est inférieure à un volume seuil minimal, empêchant ainsi l'activation du dispositif de chauffage électrique (119) .

10. Système de génération d'aérosol à fonctionnement électrique selon l'une quelconque des revendications 1 à 9, comprenant en outre un capteur de température pour mesurer la température de l'au moins un élément de chauffage et dans lequel les circuits électriques sont disposés pour commander la température de l'au moins un élément de chauffage tel que détectée par le capteur de température et déterminer l'épuisement du substrat formant aérosol liquide (115) chauffé par le dispositif de chauffage sur la base de la température telle que détectée par le capteur de température.

11. Système de génération d'aérosol à fonctionnement électrique selon l'une quelconque des revendications 1 à 10, dans lequel les circuits électriques (109) sont disposés pour mesurer la résistance électrique de l'au moins un élément de chauffage, pour établir la température de l'élément de chauffage à partir de la résistance électrique mesurée.

12. Système de génération d'aérosol à fonctionnement électrique selon la revendication 11, dans lequel les circuits électriques (109) sont disposés pour mesurer la résistance électrique de l'au moins un élément de chauffage par la mesure du courant traversant l'au moins un élément de chauffage et la tension traversant l'au moins un élément de chauffage et par la détermination de la résistance électrique de l'au moins un élément de chauffage à partir du courant et de la tension mesurés.

13. Procédé comprenant :
la fourniture d'un système de génération d'aérosol à fonctionnement électrique comprenant une partie de stockage de liquide pour stocker un substrat formant aérosol liquide (115) et un dispositif de chauffage électrique (119) comprenant au moins un élément de chauffage pour chauffer le substrat formant aérosol liquide ;
l'activation automatique du dispositif de chauffage électrique à un moment particulier pendant une durée d'activation automatique dans une période d'inactivité du dispositif de chauffage électrique ; et
la détermination de l'épuisement du substrat formant aérosol liquide chauffé par le dispositif de chauffage électrique sur la base d'une relation entre une puissance appliquée à l'élément de chauffage et une variation de température résultante de l'élément de chauffage.

14. Circuits électriques pour un système de génération d'aérosol à fonctionnement électrique comprenant une partie de stockage de liquide pour stocker un substrat formant aérosol liquide (115) et un dispositif de chauffage électrique (119) comprenant au moins un élément de chauffage pour chauffer le substrat formant aérosol liquide, les circuits électriques (109) étant disposés pour réaliser le procédé selon la revendication 13.

15. Support de stockage lisible par ordinateur ayant stocké sur celui-ci un programme informatique qui, lorsqu'il fonctionne sur un circuit électrique programmable pour un système de génération d'aérosol à fonctionnement électrique, le système de génération d'aérosol à fonctionnement électrique comprenant une partie de stockage de liquide pour stocker un substrat formant aérosol liquide (115) et un dispositif de chauffage électrique (119) comprenant au moins un élément de chauffage pour chauffer le substrat formant aérosol liquide, amène le circuit électrique programmable à réaliser le procédé selon la revendication 13.
